Europäisches Patentamt

European Patent Office

Office européen des brevets

(11) Veröffentlichungsnummer: **0 064 218**

**A2**

(12) # EUROPÄISCHE PATENTANMELDUNG

(21) Anmeldenummer: **82103340.4**

(22) Anmeldetag: **21.04.82**

(51) Int. Cl.³: **C 01 D 3/02**

(30) Priorität: **28.04.81 DE 3116690**

(43) Veröffentlichungstag der Anmeldung:
**10.11.82 Patentblatt 82/45**

(84) Benannte Vertragsstaaten:
**FR GB IT NL**

(71) Anmelder: **Rohde & Schwarz GmbH & Co. KG**
**Mühldorfstrasse 15**
**D-8000 München 80(DE)**

(72) Erfinder: **Reichel, Thomas**
**Kreillerstrasse 126**
**D-8000 München 82(DE)**

(72) Erfinder: **Betz, Tilman**
**Schlehenweg 3**
**D-8031 Gröbenzell(DE)**

(72) Erfinder: **Köhler, Dieter**
**Annabrunnerstrasse 4**
**D-8000 München 80(DE)**

(72) Erfinder: **Schermer, Josef**
**Stellastrasse 14**
**D-8070 Ingolstadt(DE)**

(74) Vertreter: **Graf, Walter**
**Sckellstrasse 1**
**D-8000 München 80(DE)**

(54) Einrichtung zum Messen von physikalischen Grössen.

(57) Bei einer Einrichtung zum Messen von physikalischen Grössen mit einem Messfühler (1) zum Umwandeln der Messgrösse in eine analoge elektrische Grösse, die über einen Mikroprozessor (10) in einem Auswertgerät (4) auswertbar ist, ist dem Messfühler (1) unverlierbar ein programmierbarer Digitalspeicher (5) zugeordnet, in welchem die Daten der Istwert-Kennlinie (J) und/oder der Istwert-Sollwert-Kennlinienabweichung (d) dieses Messfühlers (1) eingespeichert ist; die Daten dieses Digitalspeichers (5) werden bei der Auswertung der elektrischen Grösse im Mikroprozessor (10) entsprechend berücksichtigt.

Fig. 1

**Patentanwalt Dipl.-Ing. Walter Graf**

0064218

O-8000 München 80
Sckellstraße 1
Telefon (089) 4 48 08 67
Telex 523703 (rus d)
Postscheck München 182826-804
Hypo-Bank München 3 860 043 210
Deutsche Bank München 53 103 797

zugelassen beim Europäischen Patentamt – admitted to the European Patent Office – Mandataire aggréé auprès l' Office Européen des Brevets

1000 – EU

- 1 -

Einrichtung zum Messen von physikalischen Grössen

Die Erfindung betrifft eine Einrichtung zum Messen von physikalischen Grössen laut Oberbegriff des Hauptanspruches.

Einrichtungen dieser Art zum Messen der verschiedenartigsten physikalischen Grössen, beispielsweise zum Messen der Temperatur, des Druckes, von Hochfrequenzspannungen oder Hochfrequenzströmen, Hochfrequenzleistungen und dergleichen. Der Messfühler wandelt die physikalische Messgrösse in eine analoge elektrische Grösse um, beispielsweise in eine Gleichspannung, einen Gleichstrom, einen Widerstandswert, eine bestimmte Ausgangsfrequenz, in eine bestimmte Phasenlage einer Wechselspannung oder dergleichen. Diese analoge elektrische Grösse wird durch eine geeignete Aufbereitungsschaltung in einem Auswertgerät in eine entsprechende Digitalgrösse umgewandelt, die dann in einem Mikroprozessor in diesem Auswertgerät entsprechend ausgewertet wird, beispielsweise als Messgrösse angezeigt wird oder für einen Regel- oder Steuerzweck weiter benutzt wird. Die Istwert-Kennlinie solcher Messfühler, d.h. der Zusammenhang zwischen der physikalischen Messgrösse und der erzeugten analogen elektrischen Grösse, ist stark von den jeweils verwendeten Bauelementen des Messfühlers abhängig und für jeden Messfühler spezifisch, die Istwert-Kennlinie weicht in der Praxis mehr oder weniger stark von der vorgegebenen Sollwert-Kennlinie ab. Dadurch entstehen Fehler, die bisher entweder toleriert wurden oder die durch einstellbare bzw. ausgesuchte Bauelemente im Messfühler bzw. im Auswertgerät so klein wie möglich gehalten wurden. Ein Austausch eines bestimmten Messfühlers an einem Auswertgerät gegen einen anderen Messfühler war nur dann möglich, wenn die jeweilige Istwert-Kennlinie des Messfühlers entweder sehr genau mit der für das Gerät charakteristischen Sollwert-Kennlinie übereinstimmt oder wenn der Messfühler abgleichbar ist. Ersteres ist in der Praxis nicht immer erreichbar, die zweite Möglichkeit des Abgleiches ist sehr umständlich und ungenau.

Es ist Aufgabe der Erfindung, eine Messeinrichtung der eingangs erwähnten Art zu schaffen, bei der diese Nachteile vermieden sind und bei der bei der Auswertung der Messgrösse automatisch die jeweilige Istwert-Kennlinie des Messfühlers berücksichtigt wird.

Diese Aufgabe wird ausgehend von einer Einrichtung laut Oberbegriff des Hauptanspruches durch dessen kennzeichnende Merkmale gelöst. Vorteilhafte Weiterbildungen ergeben sich aus den Unteransprüchen.

Nach der Erfindung ist jedem einzelnen Messfühler ein einfacher Festwertspeicher unverlierbar zugeordnet, beispielsweise ein sogenanntes einmal programmierbares PROM oder ein unter gewissen Bedingungen nachprogrammierbares EPROM, und zwar ist dieser Speicher entweder unmittelbar im Messfühler selbst eingebaut oder in der Zuleitung zum Auswertgerät in einem gesonderten Gehäuse untergebracht. Der Speicher kann auch in dem Verbindungselement zwischen Messfühler und Auswertgerät untergebracht sein. Wesentlich ist, dass der Speicher jeweils unverlierbar einem bestimmten Messfühler zugeordnet ist, also mit diesem unverlierbar mechanisch verbunden ist. Im einfachsten Fall sind der Messführer und der zugeordnete Digitalspeicher besonders gekennzeichnet, also beispielsweise mit einer gleichartigen Markierung versehen, die sicherstellt, dass nur diese so gekennzeichneten Teile zusammengehören. Am Messfühler könnte auch ein spezieller Stecker angebracht sein, in den nur der Stecker eines zugehörigen Digitalspeichers passt. Auch eine reine mechanische Verbindung von Messfühler und zugeordnetem Dititalspeicher, beispielsweise durch ein Bändchen oder ein Kettchen, ist denkbar. In den zugeordneten Speicher, der beim Anschliessen des Messfühlers an das Auswertgerät ebenfalls mit diesem verbunden wird, können dann in bekannter Weise über ein geeignetes Programmiergerät in einem einmaligen Kalibriervorgang Daten der Istwert-Kennlinie oder Daten der Istwert-Sollwert-Kennlinienabweichung des zugehörigen Messfühlers in diesen Speicher eingespeichert werden, die während des Messvorganges über das Auswertgerät aus diesem Speicher wieder ausgelesen werden und bei der Auswertung der vom Messfühler gelieferten elektrischen Grösse entsprechend berücksichtigt werden. Es genügt im allgemeinen nur einige wenige ausgewählte Punkte der Kennlinie einzuspeichern, ausdenen dann durch Interpolation die gesamten Kennliniendaten oder das gesamte Kennlinienfeld rückgewonnen werden können. Durch den Kalibriervorgang werden also beispielsweise einige Punkte der Istwert-Kennlinie des Messfühlers in den Speicher eingespeichert, anschliessend kann dann bei einem Messvorgang über das Auswertgerät bestimmt werden, welcher Eingangsgrösse auf der Istwert-Kennlinie die vom Messfühler gelieferte analoge elektrische Messgrösse entspricht. Auf diese Weise wird über das Auswertgerät der richtige Messwert ermittelt und angezeigt. Im Speicher sind also in diesem Fall jeweils Wertpaare der Kennlinie gespeichert, die für den jeweiligen Messfühler die Zuordnung zwischen der erzeugten analogen elektrischen Grösse und der normierten Eingangsgrösse darstellen. Aus Gründen der Speicherkapazität kann es vorteilhaft sein, im Speicher jeweils nur die Istwert-Sollwert-Abweichungen abzuspeichern und beim Messvorgang abzufragen, in diesem Fall muss dann im Auswertgerät zusätzlich noch unter Berücksichtigung der Sollwert-Kennlinie aus diesen Werten die eigentliche Istwert-Kennlinie rückgewonnen werden. Nach der Erfindung können auch für verschiedene Messparameter gesonderte Kennliniendaten im Speicher eingespeichert werden, die dann von Hand oder automatisch beim Messvorgang ausgelesen und berücksichtigt werden. Die Daten der jeweiligen Istwert-Kennlinie des Messfühlers sind also nach der Erfindung für jeden einzelnen Messfühler gespeichert und vom Auswertgerät abfragbar und es ist daher möglich, solche Messfühler gegeneinander auszutauschen, teure und komplizierte Abgleicharbeiten werden daher überflüssig, es können auch grob tolerierte Bauteile im Messfühler verwendet werden. Im Speicher können auch noch andere für den Messfühler spezifische Daten eingespeichert sein, die dann für das Auswertgerät zur Verfügung stehen, beispielsweise Teilungsfaktoren bei Hochfrequenz-Spannungsmessköpfen mit integrierten Spannungsteilern, Herstellungsdaten und dergleichen. Die erfindungsgemässe Massnehme ist für die verschiedenartigsten Messfühler geeignet, mit denen die eingangs erwähnten verschiedenartigsten physikalischen Messgrössen in entsprechende verschiedenartige analoge elektrische Ausgangsgrössen umgewandelt werden.

Die Erfindung wird im folgenden anhand schematischer Zeichnungen an einem Ausführungsbeispiel näher erläutert.

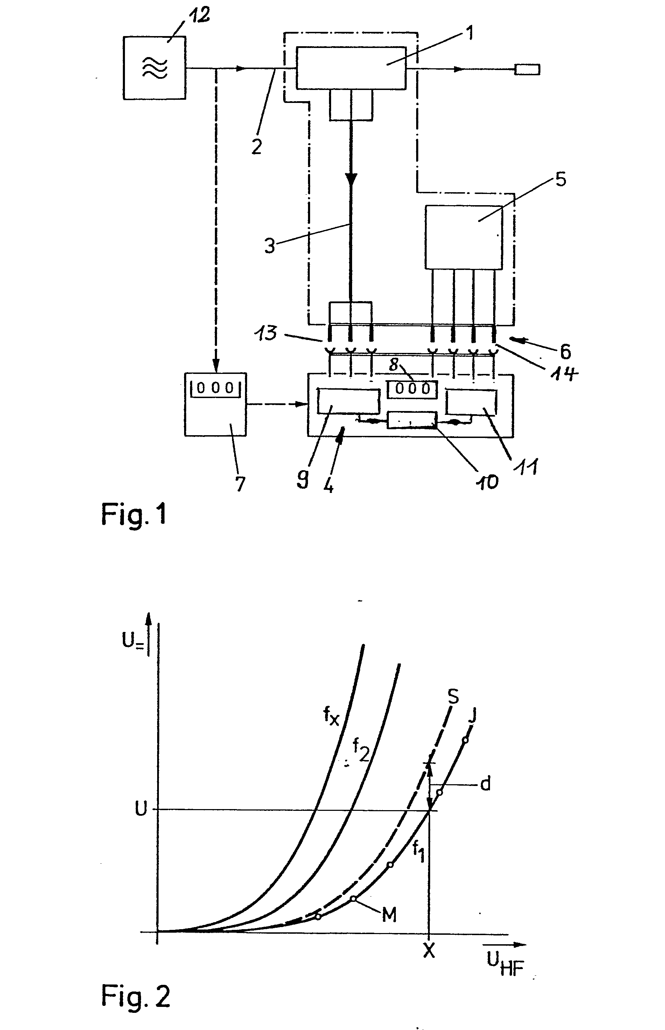

Fig. 1 zeigt das Prinzipschaltbild einer Messeinrichtung nach der Erfindung mit einem Messfühler 1 zum Messen der Hochfrequenzspannung auf einer Hochfrequenzleitung 2, die über einen Hochfrequenzgenerator 12 gespeist ist.

Die Hochfrequenzspannung wird durch den Messfühler 1 in eine analoge Gleichspannung umgewandelt, die über eine Leitung 3 und die Kontakte 13 einer Steckverbindung 6 einem Auswertgerät 4 zugeführt und dort weiterverarbeitet wird. Der Messfühler 1 ist mit einem elektronischen Digitalspeicher 5 mechanisch zusammengebaut. Dieser Speicher 5 wird über die schematisch angedeuteten Verbindungsleitungen und Verbindungskontakte 14 beim Anschliessen des Messfühlers mit dem Auswertgerät 4 elektrisch verbunden und über das Auswertgerät 4 kann dieser Speicher ausgelesen werden.

Das Auswertgerät 4 enthält einen Mikroprozessor 10, dem unter anderem in bekannter Weise zusätzliche nicht näher dargestellte Speicher zugeordnet sind. Die vom Messfühler 1 über die Leitung 3 zugeführte analoge Gleichspannung wird durch eine schematisch angedeutete Aufbereitungsschaltung 9 in ein entsprechendes Digital-Signal umgewandelt, das dann im Mikroprozessor 10 entsprechend ausgewertet wird. Das Ergebnis wird beispielsweise über eine Anzeigeeinrichtung 8 als Messwert angezeigt.

Der Inhalt des Digitalspeichers 5 kann auf die verschiedenste Weise dem Mikroprozessor 10 zugeführt werden. Im einfachsten Fall ist zwischen dem Speicher 5 und dem Mikroprozessor 10 eine entsprechende Vielfachverbindung über den Mehrfachstecker 6 vorgesehen, so dass der Mikroprozessor 10 den Inhalt des Speichers 5 unmittelbar genauso bei der Auswertung berücksichtigen kann wie dies mit dem Inhalt der anderen nicht dargestellten Speichern geschieht. Um eine solche Mehrfach-Leitungsverbindung zwischen Auswertgerät 4 und Speicher 5 zu vermeiden, könnte auch eine schematisch angedeutete zusätzliche Leseeinrichtung 11 vorgesehen sein, mit der der Inhalt des Speichers 5 beispielsweise seriell über nur wenige Leitungen in den Mikroprozessor 10 eingelesen wird.

Diese Ausleseeinrichtung 11 kann hierbei entweder wie gezeigt im Gerät 4 untergebracht sein, u.U. ist es auch vorteilhaft, diese Ausleseeinrichtung 11 oder zumindest einen Teil davon in der aus Messfühler 1 und Speicher 5 bestehenden Baueinheit unterzubringen. Dadurch kann die Anzahl der nötigen Verbindungsleitungen zwischen Speicher 5 und Auswertgerät 4 noch weiter reduziert werden.

Angenommen, der Messfühler 1 besitzt infolge der verwendeten Bauelemente die in Fig. 2 dargestellte Istwert-Kennlinie J, die von der eigentlichen Sollwert-Kennlinie S wie dargestellt abweicht. Durch einen Kalibriervorgang können die Kennliniendaten dieser Istwert-Kennlinie J festgestellt und in den programmierbaren Speicher 5 eingelesen werden. Jedem Hochfrequenz-Eingangsspannungswert X ist im Sinne Fig. 2 ein jeweiliger Ausgangs-Gleichspannungswert U zugeordnet. Beim Kalibriervorgang können auf diese Weise beispielsweise die in Fig. 2 eingezeichneten fünf ausgewählten Messpunkte M eingespeichert werden, d.h. im Speicher 5 wird zunächst für einen ersten vorbestimmten Messwert X1 (beispielsweise 10 mV Hochfrequenzspannung) der zugehörige Istwert U1 eingespeichert, für den nächsten Eingangsspannungswert X2 (beispielsweise 100 mV) der zugehörige Ausgangs-Istwert U2 usw. Diese die Istwert-Kennlinie charakterisierenden Wertpaare U/X stehen dann während des Messvorganges für das Auswertgerät 4 zur Verfügung. Wird anschliessend eine unbekannte Messspannung über den Messfühler 1 auf der Leitung 2 abgetastet und liefert damit die Leitung 3 eine Ausgangsgleichspannung U, so wird im Mikroprozessor des Auswertgerätes 4 aus den im Speicher 5 eingespeicherten Istwert-Kennliniendaten J festgestellt, dass diesem Gleichspannungswert U der Hochfrequenz-Spannungswert X entspricht, der dann angezeigt wird.

Aus Speicherkapazitätsgründen kann es vorteilhaft sein, im Speicher 5 nur die sich aus Fig. 2 ergebenden Sollwert-Istwert-Kennlinienabweichungen d und in einem dem Mikroprozessor 10 zugeordneten Speicher im Auswertgerät die vorgegebene Sollwert-Kennlinie zu speichern. Während eines Messvorganges können dann aus den im Auswertgerät 5 eingespeicherten Sollwert-Kennliniendaten S unter Berücksichtigung der aus dem Speicher 5 ausgelesenen Differenzwerte d die gewünschten Istwert-Kennliniendaten J rückgewonnen werden, mit denen dann wie oben beschrieben wieder die eigentliche Anzeigegrösse ermittelt wird.

Bei der Kalibrierung können natürlich auch für verschiedene Frequenzen unmittelbar die zugehörigen unterschiedlichen Istwert-Kennlinien eingespeichert werden, wie dies in Fig. 2 für die Frequenzen f1, f2, fx angedeutet ist. Natürlich können auch hier wieder die diesen einzelnen Frequenzen zugeordneten Sollwert-Istwert-Abweichungen eingespeichert sein. Ist dem Benutzer bekannt, dass er eine Hochfrequenzspannungsmessung im Bereich der Frequenz f1 durchzuführen hat, so kann er beispielsweise durch eine entsprechende Eingabetastatur am Auswertgerät 4 die der Frequenz f1 zugehörige Istwert-Kennlinie aus dem Speicher 5 auslesen und bei der Auswertung entsprechend berücksichtigen. Dieser Vorgang kann gegebenenfalls auch automatisiert werden, wenn die jeweilige Messfrequenz bekannt ist, sei es als vorgegebene Einstellung am die Leitung 2 speisenden Generator 12 oder sei es durch einen zusätzlichen Frequenzmessvorgang. In dem gezeigten Ausführungsbeispiel ist dargestellt, wie durch einen für solche Hochfrequenzspannungsmessungen meist sowieso vorgesehenen Frequenzmesser 7 die jeweilige Messfrequenz festgestellt wird und wie über den Frequenzmesser 7 dann automatisch das Auswertgerät 4 entsprechend gesteuert wird. Im Auswertgerät wird die zugeführte Frequenzinformation automatisch ausgewertet und es wird dann automatisch die zugehörige Kennlinie aus dem
Speicher 5 ausgelesen und bei der jeweiligen Messung
berücksichtigt.

Das Auslesen der im Speicher 5 gespeicherten Kennliniendaten in das Auswertgerät kann in einem einmaligen Auslesevorgang beispielsweise beim Einschalten des Geräts
durchgeführt und dann für die verschiedenen Messungen
beibehalten werden. Die Auslesung der Kennliniendaten
kann jedoch auch kontinuierlich während des eigentlichen
Messvorganges erfolgen.

Patentanwalt Dipl.-Ing. Walter Graf

D-8000 München 80
Sckellstraße 1
Telefon (089) 48 06 54
Telex 523703 (rus d)
Postscheck München 182826-804
Hypo-Bank München 3860043210
Deutsche Bank München 53103797

**0064218**

zugelassen beim Europäischen Patentamt – admitted to the European Patent Office – Mandataire aggréé auprès l' Office Européen des Brevets

1000- EU

ROHDE & SCHWARZ GmbH & Co KG, 8000 München 80

Patentansprüche

1. Einrichtung zum Messen von physikalischen Grössen mit einem Messfühler zum Umwandeln der Messgrösse in eine analoge elektrische Grösse, der an ein Auswertgerät anschliessbar ist, in welchem die elektrische Grösse über einen Mikroprozessor auswertbar ist, d a d u r c h   g e k e n n z e i c h n e t, dass dem Messfühler (1) unverlierbar ein programmierbarer Digitalspeicher (5) zugeordnet ist, in welchen die Daten der Istwert-Kennlinie (J) und/oder der Istwert-Sollwert-Kennlinienabweichung (d) des zugehörigen Messfühlers (1) einspeicherbar sind, und welcher an den Mikroprozessor (10) des Auswertgerätes (4) anschliessbar ist und mit diesem so zusammenwirkt, dass die Daten dieses Digitalspeichers (5) bei der Auswertung der vom Messfühler (1) an den Mikroprozessor (10) gelieferten elektrischen Grösse entsprechend berücksichtigt werden.

2. Einrichtung nach Anspruch 1, d a d u r c h   g e - k e n n z e i c h n e t, dass der Digitalspeicher (5) mit dem Messfühler (1) unverlierbar zu einer Einheit zusammengebaut ist und Messfühler (1) und Digitalspeicher (5) über einen Mehrfachstecker (6) an das Auswertgerät (4) anschliessbar sind.

3. Einrichtung nach Anschluss 1 oder 2, d a d u r c h g e k e n n z e i c h n e t, dass im Auswertwerät (4) und/oder in der Messfühler-Digitalspeicher-Einheit (1,5) eine Einrichtung zum Auslesen des Speicherinhaltes in den Mikroprozessor (10) vorgesehen ist.

4. Einrichtung nach einem der vorhergehenden Ansprüche, d a d u r c h g e k e n n z e i c h n e t, dass im Digitalspeicher (5) Kennliniendaten für verschiedene Messparameter (f1,f2,fx) gesondert gespeichert sind.

5. Einrichtung nach Anspruch 4 zum Umwandeln einer zu messenden Hochfrequenzspannung in eine auszuwertende Gleichspannung, d a d u r c h g e k e n n z e i c h n e t, dass im digitalen Speicher (5) die Kennliniendaten für verschiedene Frequenzen (fa,f2,fx) gesondert gespeichert sind.

6. Einrichtung nach Anspruch 4 oder 5, d a d u r c h g e k e n n z e i c h n e t, dass die den verschiedenen Messparametern (f1,f2,fx) zugeordneten Kennliniendaten gesondert aus dem Digitalspeicher (5) auslesbar und im Mikroprozessor .(10) berücksichtigbar sind.

7. Einrichtung nach einem der Ansprüche 4 bis 6, d a d u r c h g e k e n n z e i c h n e t, dass dem Auswertgerät (4) von aussen Informationen über die bei den einzelnen Messvorgängen geltenden Messparameter (f1,f2, fx) zuführbar sind (aus Frequenzmesser 7) und über diese Informationen die Ausleseeinrichtung (11) so steuerbar ist, dass aus dem Digitalspeicher (5) automatisch jeweils die dem momentanen Messvorgang zugeordneten Kennliniendaten ausgelesen und im Mikroprozessor (10) berücksichtigt werden.

Fig. 1

Fig. 2